# EUROPÄISCHE PATENTSCHRIFT

(11) **EP 4 593 762 B1**
(45) Veröffentlichungstag und Bekanntmachung des Hinweises auf die Patenterteilung: **04.02.2026**
(21) Anmeldenummer: 23764846.4
(22) Anmeldetag: 29.08.2023
(51) Int. Cl.: A61F 2/16, A61F 2/00

(54) **INJEKTOR MIT ZWEI TEILINJEKTORKÖRPERN UND ZWEI TEILKOLBEN UND VERPACKUNG MIT DEM INJEKTOR**
INJECTOR WITH TWO PARTIAL INJECTOR BODIES AND TWO PARTIAL PLUNGERS, AND PACKAGING WITH THE INJECTOR
INJECTEUR À DEUX CORPS D'INJECTEUR PARTIELS ET DEUX PISTONS PARTIELS, ET EMBALLAGE AVEC L'INJECTEUR

(30) Priorität: 26.09.2022 DE 102022124678
(43) Veröffentlichungstag der Anmeldung: 06.08.2025
(73) Patentinhaber: Carl Zeiss Meditec AG, 07745 Jena (DE)
(72) Erfinder: BADUR, Thorben, 73447 Oberkochen (DE); SCHARSICH, Christina, 73447 Oberkochen (DE); MASCH, Jennifer-Magdalena, 73447 Oberkochen (DE); MOEIN, Hadi, 73447 Oberkochen (DE)
(74) Vertreter: PATERIS Patentanwälte PartmbB
(86) Internationale Anmeldenummer: PCT/EP2023/073717
(87) Internationale Veröffentlichungsnummer: WO 2024/068166

(56) Entgegenhaltungen:
- DE-B3- 102021 116 615
- US-A1- 2020 054 833

## Beschreibung

Die Erfindung betrifft einen Injektor mit einem ersten Teilinjektorkörper und einem zweiten Teilinjektorkörper sowie einem ersten Teilkolben und einem zweiten Teilkolben. Zudem betrifft die Erfindung eine Verpackung mit dem Injektor.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges üblicherweise mittels Phakoemulsifikation zerkleinert und anschließend aus dem Kapselsack des Auges abgesaugt. Danach wird eine Intraokularlinse mittels des Injektors in das Auge eingesetzt. Der Injektor wird herkömmlich in einer sterilen Verpackung geliefert, wobei die Verpackung frühestens kurz vor der Kataraktbehandlung geöffnet wird. Die Verpackung ist danach zu entsorgen. Dabei fällt jedoch nachteilig eine große Menge an Abfall an. DE 10 2020 115 489 A1 offenbart eine Injektoranordnung für ein Einführen einer Intraokularlinse. DE 600 03 908 T2 offenbart eine Haltevorrichtung für Intraokularlinsen und Injektionsvorrichtungen sowie Verfahren zu ihrer Verwendung. DE 10 2021 116 615 B3 offenbart einen Injektor mit einem ersten Kolben und einem zweiten Kolben. Aufgabe der Erfindung ist es daher, einen Injektor für ein Einführen einer Intraokularlinse in den Kapselsack eines Auges und eine Verpackung mit dem Injektor zu schaffen, bei denen nur eine geringe Menge an Abfall anfällt.

Der erfindungsgemäße Injektor für ein Einführen einer Intraokularlinse in den Kapselsack eines Auges weist einen Injektorkörper, einen Kolben und ein Gelenk auf. Der Kolben ist in einer Einführrichtung, in der die Intraokularlinse zum Einführen in den Kapselsack aus dem Injektor heraus zu verlagern ist, längsverlagerbar in dem Injektorkörper angeordnet. Der Injektorkörper weist einen ersten Teilinjektorkörper, der eine Spitze mit einer Spitzenöffnung aufweist, und einen zweiten Teilinjektorkörper auf, der ein proximales Ende des Injektorkörpers aufweist und der mittels des Gelenks verschwenkbar an dem ersten Teilinjektorkörper angebracht ist, wodurch der Injektorkörper in einen Gebrauchszustand, in dem das proximale Ende der Spitzenöffnung abgewandt angeordnet ist, und einen Lagerungszustand verschwenkbar ist, in dem der Injektorkörper in der Einführrichtung eine kürzere Erstreckung als in dem Gebrauchszustand hat. Durch ein Verschwenken des proximalen Endes weg von der Spitzenöffnung ist der Injektorkörper von dem Lagerungszustand in den Gebrauchszustand bringbar. Der Kolben weist einen ersten Teilkolben, der längsverlagerbar an dem ersten Teilinjektorkörper angeordnet ist, und einen zweiten Teilkolben auf, der in dem Lagerungszustand beabstandet von dem ersten Teilkolben angeordnet ist und der in dem Gebrauchszustand eingerichtet ist, durch ein Längsverlagern des zweiten Teilkolbens in der Einführrichtung den ersten Teilkolben in der Einführrichtung zu verlagern.

Indem der Injektor mit dem Injektorkörper in dessen Lagerungszustand in einer Verpackung angeordnet wird, kann die Verpackung mit einem anderen Oberfläche zu Volumen Verhältnis als bei einer herkömmlichen Verpackung mit einem herkömmlichen Injektor vorgesehen werden, dessen Injektorkörper nicht von dem Lagerungszustand in den Gebrauchszustand verschwenkbar ist. Beispielsweise kann in einem Fall, dass das Volumen der Verpackung gleich dem Volumen der herkömmlichen Verpackung ist, die Oberfläche der Verpackung kleiner als die Oberfläche der herkömmlichen Verpackung ausgeführt werden. In einem Vergleichsbeispiel können die Verpackung und die herkömmliche Verpackung jeweils die Form eines Quaders haben, wobei eine Höhe des Quaders in die Einführrichtung, eine Breite des Quaders quer zu der Einführrichtung und eine Tiefe des Quaders senkrecht zu der Höhe und senkrecht zu der Breite orientiert sind. In dem Vergleichsbeispiel beträgt für die herkömmliche Verpackung die Höhe 5 w.E. (willkürliche Einheiten), die Breite 2 w.E. und die Tiefe 1 w.E.. Bei einem gleichen Volumen bei der Verpackung wie bei der herkömmlichen Verpackung beträgt bei der Verpackung die Höhe 3 w.E., die Breite 2/0,6≈3,33 w.E. und die Tiefe 1 w.E.. In diesem Vergleichsbeispiel beträgt die Oberfläche der Verpackung 96 % der Oberfläche der herkömmlichen Verpackung.

Der Injektorkörper weist bevorzugt einen Einrastmechanismus auf, der eingerichtet ist, in dem Gebrauchszustand einzurasten und somit ein Verschwenken des Injektorkörpers aus dem Gebrauchszustand heraus arretiert. Dadurch kann vorteilhaft ein Verschwenken des Injektorkörpers aus dem Gebrauchszustand heraus, insbesondere während die Intraokularlinse in den Kapselsack eingeführt wird, vermieden werden.

Es ist bevorzugt, dass der Injektor einen Faltkeil aufweist, der eingerichtet ist, die Intraokularlinse zu falten, bevor die Intraokularlinse aus dem Injektor heraus verlagert wird.

Dabei ist besonders bevorzugt, dass der Faltkeil derart mit dem zweiten Teilinjektorkörper und/oder mit dem zweiten Teilkolben gekoppelt ist, dass durch ein Verschwenken des proximalen Endes aus dem Lagerungszustand heraus und hin zu der Spitzenöffnung der Faltkeil die Intraokularlinse faltet. Dabei kann der Faltkeil an dem zweiten Teilkolben angebracht sein und von dem zweiten Teilkolben vorstehen und/oder der Faltkeil kann an dem zweiten Teilinjektorkörper angebracht sein und von dem zweiten Teilinjektorkörper vorstehen.

Alternativ ist es besonders bevorzugt, dass der Faltkeil derart mit dem zweiten Teilinjektorkörper und/oder mit dem zweiten Teilkolben gekoppelt ist, dass durch ein Verschwenken des proximalen Endes aus dem Lagerungszustand heraus und weg von der Spitzenöffnung der Faltkeil die Intraokularlinse faltet. Der Injektor kann ein Zugelement aufweisen, das an dem zweiten Teilkolben und/oder an dem zweiten Teilinjektorkörper befestigt ist und an dem Faltkeil befestigt ist und mittels dem der Faltkeil mit dem zweiten Teilinjektorkörper und/oder mit dem zweiten Teilkolben gekoppelt ist. Bei dem Zugelement kann es sich beispielsweise um ein Seil oder eine Stange handeln.

Der Injektor weist bevorzugt die Intraokularlinse auf, die in dem ersten Injektorkörper und in der Einführrichtung zwischen dem ersten Teilkolben und der Spitzenöffnung angeordnet ist.

Es ist bevorzugt, dass der Injektor eingerichtet ist, die Intraokularlinse mit einem Fluid zu benetzen, wenn der Injektorkörper von dem Lagerungszustand in den Gebrauchszustand verschwenkt wird. Indem die Intraokularlinse mit dem Fluid benetzt wird, kann eine Beschädigung der Intraokularlinse vermieden werden, wenn die Intraokularlinse von dem Kolben verlagert wird. Dadurch, dass es erforderlich ist, vor dem Einführen der Intraokularlinse in den Kapselsack den Injektorkörper in den Gebrauchszustand zu verschwenken, wird die Intraokularlinse automatisch benetzt. Bei dem Fluid kann es sich beispielsweise um opthalmisches Viskoelastikum (englisch: opthalmic viscoelastic device, OVD) oder um eine physiologische Kochsalzlösung handeln.

Es ist bevorzugt, dass der erste Teilinjektorkörper eine Kammer aufweist, in der das Fluid angeordnet ist und die eine Kammeröffnung aufweist, wobei der zweite Teilinjektorkörper und/oder der zweite Teilkolben eingerichtet ist, das Fluid via die Kammeröffnung aus der Kammer zu drücken, indem der Injektorkörper von dem Lagerungszustand in den Gebrauchszustand verschwenkt wird. Indem das Fluid aus der Kammer gedrückt wird, kann die Intraokularlinse von dem Fluid benetzt werden. Der Injektor weist bevorzugt einen Kammerkolben auf, der längsverlagerbar in der Kammer angeordnet ist. Das Fluid ist bevorzugt in dem Lagerungszustand in der Einführrichtung zwischen dem Kammerkolben und der Kammeröffnung angeordnet. Der zweite Teilinjektorkörper und/oder dem zweiten Teilkolben kann den Kammerkolben kontaktieren und zu der Kammeröffnung hin verlagern, wenn der Injektorkörper von dem Lagerungszustand in den Gebrauchszustand verschwenkt wird, wodurch der Kammerkolben das Fluid aus der Kammeröffnung drückt.

Der Injektor weist bevorzugt eine Arretierungsvorrichtung auf, die einen Arretierungszustand, in dem die Arretierungsvorrichtung ein Längsverlagern des ersten Teilkolbens und/oder des zweiten Teilkolben arretiert, und einen Entarretierungszustand hat, in dem die Arretierungsvorrichtung das Längsverlagern des ersten Teilkolbens und/oder des zweiten Teilkolbens ermöglicht. Durch das Vorsehen der Arretierungsvorrichtung kann beispielsweise vermieden werden, dass der erste Teilkolben und/oder der zweite Teilkolben außerhalb des Gebrauchszustands aus dem Injektorgehäuse gelangen. Es ist besonders bevorzugt, dass der Injektor eingerichtet ist, die Arretierungsvorrichtung von dem Arretierungszustand in den Entarretierungszustand zu bringen, indem der Injektorkörper von dem Lagerungszustand in den Gebrauchszustand verschwenkt. Dadurch gelangt die Arretierungsvorrichtung automatisch in den Entarretierungszustand, wodurch der Injektor besonders fehlerarm bei der Benutzung ist.

Es ist bevorzugt, dass der Injektor eingerichtet ist, dass der zweite Teilkolben den ersten Teilkolben in der Einführrichtung verlagert, wenn der Injektorkörper aus dem Lagerungszustand in den Gebrauchszustand verschwenkt wird.

Bei der erfindungsgemäßen Verpackung ist der Injektor in der Verpackung angeordnet und der Injektorkörper ist in dem Lagerungszustand angeordnet.

Im Folgenden wird anhand der beigefügten schematischen Zeichnungen die Erfindung näher erläutert. Es zeigen
Figur 1 einen Längsschnitt durch eine erste Ausführungsform des erfindungsgemäßen Injektors in einem Lagerungszustand eines Injektorkörpers des Injektors,
Figur 2 den Längsschnitt aus Figur 1 in einem Gebrauchszustand des Injektorkörpers,
Figur 3 eine Seitenansicht einer zweiten Ausführungsform des Injektors,
Figur 4 einen Längsschnitt einer dritten Ausführungsform des Injektors,
Figur 5 eine Längsschnitt einer vierten Ausführungsform des Injektors und
Figur 6 eine Draufsicht auf einer Verpackung mit einer fünften Ausführungsform des Injektors.

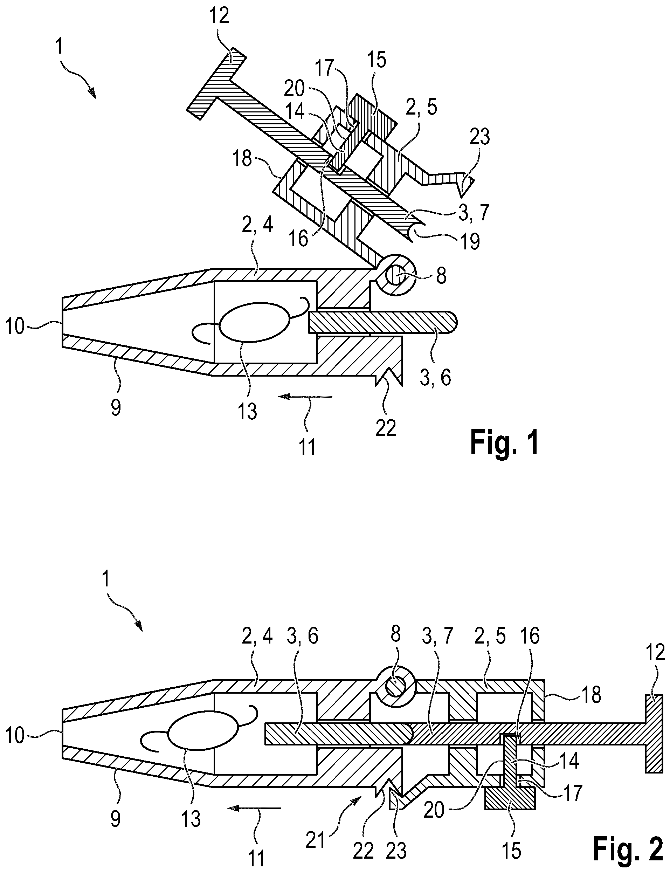

Wie es aus Figuren 1 bis 6 ersichtlich ist, weist ein Injektor 1 für ein Einführen einer Intraokularlinse 13 in den Kapselsack eines Auges einen Injektorkörper 2, einen Kolben 3 und ein Gelenk 8 auf. Der Kolben 3 ist in einer Einführrichtung 11, in der die Intraokularlinse 13 zum Einführen in den Kapselsack aus dem Injektor 1 heraus zu verlagern ist, längsverlagerbar in dem Injektorkörper 2 angeordnet. Der Injektorkörper 2 weist einen ersten Teilinjektorkörper 4, der eine Spitze 9 mit einer Spitzenöffnung 10 aufweist, und einen zweiten Teilinjektorkörper 5 auf, der ein proximales Ende 18 des Injektorkörpers 2 aufweist und der mittels des Gelenks 8 bevorzugt verschwenkbar an dem ersten Teilinjektorkörper 4 angebracht ist, wodurch der Injektorkörper 2 in einen Gebrauchszustand (siehe Figur 2), in dem das proximale Ende 18 der Spitzenöffnung 10 abgewandt angeordnet ist, und einen Lagerungszustand (siehe Figuren 1 und 3 bis 6) verschwenkbar ist, in dem der Injektorkörper 2 in der Einführrichtung 11 eine kürzere Erstreckung als in dem Gebrauchszustand hat. Durch ein Verschwenken des proximalen Endes 18 weg von der Spitzenöffnung 10 ist der Injektorkörper 2 von dem Lagerungszustand in den Gebrauchszustand bringbar. Das Gelenk 8 kann ein Lager mit einem Zapfenelement sein, das in einer Lagerhülse geführt ist. Es kann jedoch auch ein Biegefedergelenk oder Filmgelenk oder allgemein jedes Element oder jede Elementgruppe sein, welches eine gegenseitige Beweglichkeit des ersten Teilinjektorkörpers 4 zu dem zweiten Teilinjektorkörper 5 ermöglicht. Der Kolben 3 weist einen ersten Teilkolben 6, der längsverlagerbar an dem ersten Teilinjektorkörper 4 angeordnet ist, und einen zweiten Teilkolben 7 auf, der in dem Lagerungszustand beabstandet von dem ersten Teilkolben 6 angeordnet ist und der in dem Gebrauchszustand eingerichtet ist, durch ein Längsverlagern des zweiten Teilkolben 7 in der Einführrichtung 11 den ersten Teilkolben 6 in der Einführrichtung 11 zu verlagern. Die Intraokularlinse 13 kann von dem Kolben 3 in dem Gebrauchszustand des Injektorkörpers 2 via die Spitzenöffnung 10 aus dem Injektor 1 heraus verlagert werden. Der zweite Teilkolben 7 kann an dem zweiten Teilinjektorkörper 5 angebracht sein, insbesondere längsverlagerbar an dem zweiten Teilinjektorkörper 2 angebracht sein.

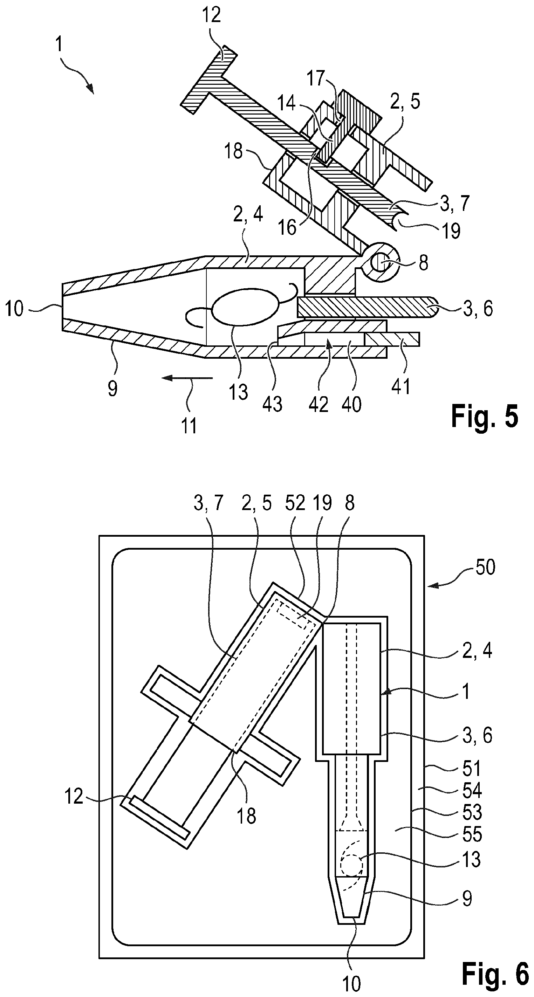

Der zweite Teilkolben 7 kann beispielsweise eingerichtet sein, den ersten Teilkolben 6 zu kontaktieren, um den ersten Teilkolben 6 in der Einführrichtung 11 zu verlagern, vergleiche Figur 2. Der erste Teilkolben 6 kann eine Kopplungsaussparung 19 aufweisen, in die das in dem Gebrauchszustand in der Einführrichtung 11 gelegene Längsende des zweiten Teilkolbens 7 eingreift, wenn der zweite Teilkolben 7 den ersten Teilkolben 6 verlagert, vergleiche Figur 6. Alternativ dazu ist denkbar, dass der zweite Teilkolben 7 eine Kopplungsaussparung 19 aufweist, in die das entgegen der Einführrichtung 11 gelegene Längsende des ersten Teilkolbens 6 eingreift, wenn der zweite Teilkolben 7 den ersten Teilkolben 6 verlagert, vergleiche Figuren 1, 2, 4 und 5. Denkbar ist am ersten Teilkolben 6 auch ein Rastelement, welches in ein Gegenstück im zweiten Teilkolben 7 einrasten kann oder auf andere Weise den ersten Teilkolben 6 mit dem zweiten Teilkolben 7 kuppelt, zum Beispiel durch Magnetkraft. Der zweite Teilkolben 7 kann an seinem Längsende, das in dem Gebrauchszustand der Spitzenöffnung 10 abgewandt angeordnet ist, eine Daumenauflage 12 aufweisen, die von einer Verdickung des zweiten Teilkolben 7 gebildet ist.

Um den Injektorkörper 2 von dem Lagerungszustand in den Gebrauchszustand zu bringen, kann ein Verschwenken des zweiten Teilinjektorkörpers 5 relativ zu dem ersten Teilinjektorkörper 4 um das Gelenk 8 von mindestens 90°, insbesondere mindestens 135° oder mindestens 165° oder mindestens 170°, erforderlich sein.

Figuren 1 und 2 zeigen, dass der Injektor 1 eingerichtet sein kann, dass der zweite Teilkolben 7 den ersten Teilkolben 6 in der Einführrichtung 11 verlagert, wenn der Injektorkörper 2 aus dem Lagerungszustand in den Gebrauchszustand verschwenkt wird. Dazu kann in dem Lagerungszustand der erste Teilkolben 6 entgegen der Einführrichtung 11 von dem ersten Teilinjektorkörper 4 vorstehen, vergleiche Figur 1. Alternativ oder zusätzlich ist es denkbar, dass der zweite Teilkolben 7 in dem Lagerungszustand über das dem proximalen Ende 18 abgewandte Ende des zweiten Teilinjektorkörpers 5 vorsteht. Es ist denkbar, dass der erste Teilinjektorkörper 4 gegenüber die Einführrichtung 11 geneigte Wände aufweist, an denen die Intraokularlinse 13 entlang gleitet, wenn der Injektorkörper 2 von dem Lagerungszustand in den Gebrauchszustand verschwenkt wird, wodurch die Intraokularlinse 13 vorgefaltet wird.

Alternativ ist denkbar, dass der erste Teilkolben 6 in dem Lagerungszustand vollständig innerhalb des ersten Teilinjektorkörpers 4 angeordnet ist und dass der zweite Teilkolben 7 in dem Lagerungszustand nicht über das dem proximalen Ende 18 abgewandte Ende des zweiten Teilinjektorkörpers 5 vorsteht. Dadurch kann eine Verpackung, in der der Injektor 1 anzuordnen ist, besonders klein ausgeführt werden.

Figuren 1 und 2 zeigen, dass der Injektorkörper 2 einen Einrastmechanismus 21 aufweisen kann, der eingerichtet ist, in dem Gebrauchszustand einzurasten und somit ein Verschwenken des Injektorkörpers 2 aus dem Gebrauchszustand heraus arretiert. Dazu können der erste Teilinjektorkörper 4 eine Einrastaussparung 22 und der zweite Teilinjektorkörper 5 einen Einrastvorsprung 23 aufweisen, der in dem Gebrauchszustand in der Einrastaussparung 22 angeordnet ist und außerhalb des Gebrauchszustands außerhalb der Einrastaussparung 22 angeordnet ist. Alternativ ist denkbar, dass der erste Teilinjektorkörper 4 einen Einrastvorsprung 23 und der zweite Teilinjektorkörper 5 eine Einrastaussparung 22 aufweisen, wobei in dem Gebrauchszustand der Einrastvorsprung 23 in der Einrastaussparung 22 angeordnet ist und außerhalb des Gebrauchszustands außerhalb der Einrastaussparung 22 angeordnet ist.

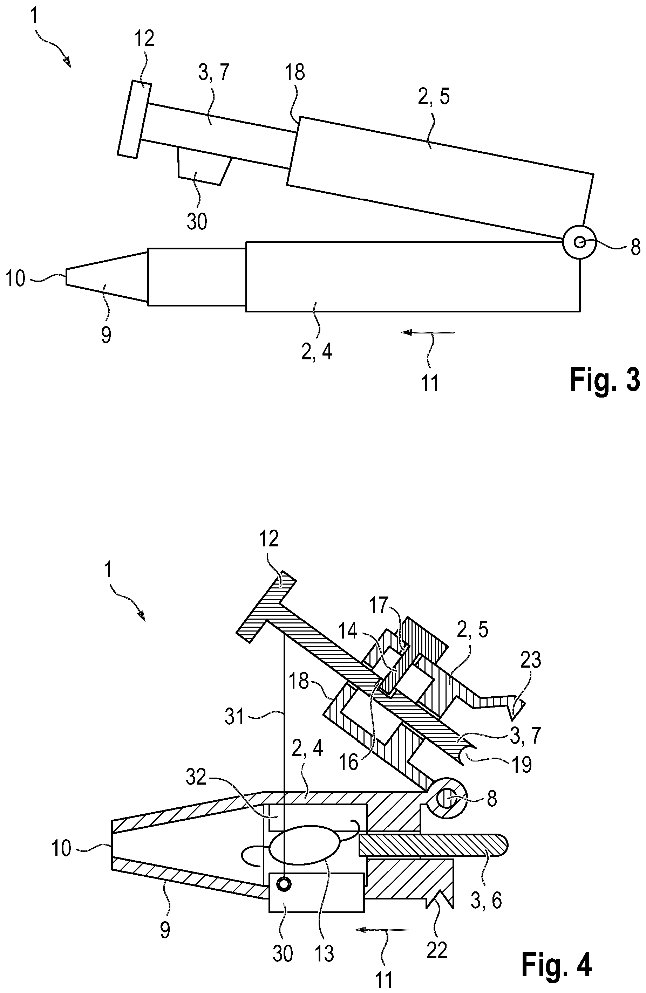

Wie es aus Figuren 3 und 4 ersichtlich ist, kann der Injektor 1 einen Faltkeil 30 aufweisen, der eingerichtet ist, die Intraokularlinse 13 zu falten, bevor die Intraokularlinse 13 aus dem Injektor 1 heraus verlagert wird. Der Faltkeil 30 kann dazu in einer Richtung verlagert werden, die quer zu der Einführrichtung 11 angeordnet ist, und der Faltkeil 30 kann dabei die Intraokularlinse 13 mittig kontaktieren und somit wölben. Figur 3 zeigt, dass der Faltkeil 30 derart mit dem zweiten Teilinjektorkörper 5 und/oder mit dem zweiten Teilkolben 7 gekoppelt sein kann, dass durch ein Verschwenken des proximalen Endes 18 aus dem Lagerungszustand heraus und hin zu der Spitzenöffnung 10 der Faltkeil 30 die Intraokularlinse 13 faltet. Beispielsweise kann der Faltkeil 30 an dem zweiten Teilkolben 7 angebracht sein und von dem zweiten Teilkolben 7 vorstehen (vergleiche Figur 3) und/oder der Faltkeil 30 kann an dem zweiten Teilinjektorkörper 5 angebracht sein und von dem zweiten Teilinjektorkörper 5 vorstehen. Figur 4 zeigt, dass der Faltkeil 30 derart mit dem zweiten Teilinjektorkörper 5 und/oder mit dem zweiten Teilkolben 7 gekoppelt sein kann, dass durch ein Verschwenken des proximalen Endes 18 aus dem Lagerungszustand heraus und weg von der Spitzenöffnung 10 der Faltkeil 30 die Intraokularlinse 13 faltet. Dazu kann der Injektor 1 ein Zugelement 31 aufweisen, das an dem zweiten Teilkolben 7 und/oder an dem zweiten Teilinjektorkörper 5 befestigt ist und an dem Faltkeil 30 befestigt ist und mittels dem der Faltkeil 30 mit dem zweiten Teilinjektorkörper 5 und/oder mit dem zweiten Teilkolben 7 gekoppelt ist. Bei dem Zugelement 31 kann es sich beispielsweise um ein Seil oder einen Stab handeln. Der erste Teilinjektorkörper 4 kann einen Anschlag 32 aufweisen, der ein Verlagern des Faltkeils 30 begrenzt. Das Zugelement 31 kann beispielsweise eingerichtet sein zu reißen, wenn der Faltkeil 30 gegen den Anschlag 32 anschlägt. Alternativ ist denkbar, dass das Zugelement 31 eingerichtet ist, von dem Faltkeil 30, dem zweiten Teilinjektorkörper 5 und/oder dem zweiten Teilkolben 7 entkoppelt zu werden, entweder von Hand oder automatisch, wenn der Faltkeil 30 gegen den Anschlag 32 anschlägt.

Figur 5 zeigt, dass der Injektor 1 eine Arretierungsvorrichtung 20 aufweisen, die einen Arretierungszustand, in dem die Arretierungsvorrichtung 20 ein Längsverlagern des ersten Teilkolbens 6 und/oder des zweiten Teilkolben 7 arretiert, und einen Entarretierungszustand hat, in dem die Arretierungsvorrichtung 20 das Längsverlagern des ersten Teilkolbens 6 und/oder des zweiten Teilkolbens 7 ermöglicht. Die Arretierungsvorrichtung 20 kann beispielsweise von einem Stift 14 gebildet sein, der sich durch ein Injektorkörperdurchgangsloch 17 hindurch erstreckt und in eine in den Kolben 3 eingebrachte Kolbenaussparung 16 eingreift. Der Stift 14 kann einen Kopf 15 aufweisen, der eine Verdickung des Stiftes 14 ist und außerhalb des Injektorkörpers 2 angeordnet ist. Durch das Vorsehen des Kopfs 15 kann der Stift 14 einfach von einer Hand angefasst werden, und somit aus der Kolbenaussparung 16 entfernt werden, wodurch das Arretierungsmittel 20 in den Entarretierungszustand gelangt. Es ist denkbar, dass der Injektor 1 eingerichtet ist, die Arretierungsvorrichtung 20 von dem Arretierungszustand in den Entarretierungszustand zu bringen, indem der Injektorkörper 2 von dem Lagerungszustand in den Gebrauchszustand verschwenkt. Dazu kann der Injektorkörper 2 beispielsweise einen Vorsprung aufweisen, der beim Verschwenken des Injektorkörpers 2 gegen den Stift 14 stößt und diesen somit zum Brechen bringt.

Der Injektor 1 kann die Intraokularlinse 13 aufweisen, die in dem ersten Injektorkörper 2 und in der Einführrichtung 11 zwischen dem ersten Teilkolben 6 und der Spitzenöffnung 10 angeordnet ist, vergleiche Figuren 1, 2, 4 und 5. Figur 5 zeigt, dass der Injektor 1 eingerichtet sein kann, die Intraokularlinse 13 mit einem Fluid 42 zu benetzen, wenn der Injektorkörper 2 von dem Lagerungszustand in den Gebrauchszustand verschwenkt wird. Dazu kann der erste Teilinjektorkörper 4 eine Kammer 40 aufweisen, in der das Fluid 42 angeordnet ist und die eine Kammeröffnung 43 aufweist, wobei der zweite Teilinjektorkörper 5 und/oder der zweite Teilkolben 7 eingerichtet ist, das Fluid 42 via die Kammeröffnung 43 aus der Kammer 40 zu drücken und somit die Intraokularlinse 13 mit dem Fluid 42 zu benetzen, indem der Injektorkörper 2 von dem Lagerungszustand in den Gebrauchszustand verschwenkt wird. Die Kammeröffnung 43 kann beispielsweise von einer Berstscheibe oder einer Membran abgedichtet sein, die eingerichtet ist zu bersten, wenn das Fluid 42 aus der Kammer 40 gedrückt wird. Der Injektor 1 kann einen Kammerkolben 41 aufweisen, der längsverlagerbar in der Kammer 40 angeordnet ist, wobei das Fluid 42 in der Einführrichtung 11 zwischen der Kammeröffnung 43 und dem Kammerkolben 41 angeordnet ist. Bei dem Fluid kann es sich beispielsweise um opthalmisches Viskoelastikum (englisch: opthalmic viscoelastic device, OVD) oder um eine physiologische Kochsalzlösung handeln.

Wie es aus Figur 6 ersichtlich ist, kann der Injektor 1 in einer Verpackung 50 angeordnet sein, wobei der Injektorkörper 2 in dem Lagerungszustand angeordnet ist. Die Verpackung 50 kann eine Basis 51 aufweisen, die insbesondere eine Vertiefung 52 aufweisen kann, in die der Injektor 1 angeordnet ist. Die Verpackung 50 kann zudem einen Deckel 53 aufweisen, der zusammen mit der Basis 51 einen Innenraum 55 der Verpackung 50 begrenzt, wobei der Injektor 1 in dem Innenraum 55 angeordnet ist. Die Basis 51 und der Deckel 53 können sich in einer vollständig umlaufenden Dichtfläche 54 kontaktieren, so dass der Innenraum 55 hermetisch abgedichtet ist. Der Deckel 53 kann transparent sein, wie es in Figur 3 beispielsweise dargestellt ist.

### Bezugszeichenliste

- 1: Injektor
- 2: Injektorkörper
- 3: Kolben
- 4: erster Teilinjektorkörper
- 5: zweiter Teilinjektorkörper
- 6: erster Teilkolben
- 7: zweiter Teilkolben
- 8: Gelenk
- 9: Spitze
- 10: Spitzenöffnung
- 11: Einführrichtung
- 12: Daumenauflage
- 13: Intraokularlinse
- 14: Stift
- 15: Kopf
- 16: Kolbenaussparung
- 17: Injektorkörperdurchgangsloch
- 18: proximales Ende
- 19: Kopplungsaussparung
- 20: Arretierungsvorrichtung
- 21: Einrastmechanismus
- 22: Einrastaussparung
- 23: Einrastvorsprung
- 30: Faltkeil
- 31: Zugelement
- 32: Anschlag
- 40: Kammer
- 41: Kammerkolben
- 42: Fluid
- 43: Kammeröffnung
- 50: Verpackung
- 51: Basis
- 52: Vertiefung
- 53: Deckel
- 54: Dichtfläche
- 55: Innenraum

## Patentansprüche

1. Injektor für ein Einführen einer Intraokularlinse (13) in den Kapselsack eines Auges, mit einem Injektorkörper (2), einem Kolben (3), der in einer Einführrichtung (11), in der die Intraokularlinse (13) zum Einführen in den Kapselsack aus dem Injektor (1) heraus zu verlagern ist, längsverlagerbar in dem Injektorkörper (2) angeordnet ist, **dadurch gekennzeichnet, dass** der Injektor (1) ein Gelenk (8) aufweist, wobei der Injektorkörper (2) einen ersten Teilinjektorkörper (4), der eine Spitze (9) mit einer Spitzenöffnung (10) aufweist, und einen zweiten Teilinjektorkörper (5) aufweist, der ein proximales Ende (18) des Injektorkörpers (2) aufweist und der mittels des Gelenks (8) verschwenkbar an dem ersten Teilinjektorkörper (4) angebracht ist, wodurch der Injektorkörper (2) in einen Gebrauchszustand, in dem das proximale Ende (18) der Spitzenöffnung (10) abgewandt angeordnet ist, und einen Lagerungszustand verschwenkbar ist, in dem der Injektorkörper (2) in der Einführrichtung (11) eine kürzere Erstreckung als in dem Gebrauchszustand hat, wobei durch ein Verschwenken des proximalen Endes (18) weg von der Spitzenöffnung (10) der Injektorkörper (2) von dem Lagerungszustand in den Gebrauchszustand bringbar ist, wobei der Kolben (3) einen ersten Teilkolben (6), der längsverlagerbar an dem ersten Teilinjektorkörper (4) angeordnet ist, und einen zweiten Teilkolben (7) aufweist, der in dem Lagerungszustand beabstandet von dem ersten Teilkolben (6) angeordnet ist und der in dem Gebrauchszustand eingerichtet ist, durch ein Längsverlagern des zweiten Teilkolbens (7) in der Einführrichtung (11) den ersten Teilkolben (6) in der Einführrichtung (11) zu verlagern.

2. Injektor gemäß Anspruch 1, wobei der Injektor (1) einen Faltkeil (30) aufweist, der eingerichtet ist, die Intraokularlinse (13) zu falten, bevor die Intraokularlinse (13) aus dem Injektor (1) heraus verlagert wird.

3. Injektor gemäß Anspruch 2, wobei der Faltkeil (30) derart mit dem zweiten Teilinjektorkörper (5) und/oder mit dem zweiten Teilkolben (7) gekoppelt ist, dass durch ein Verschwenken des proximalen Endes (18) aus dem Lagerungszustand heraus und hin zu der Spitzenöffnung (10) der Faltkeil (30) die Intraokularlinse (13) faltet.

4. Injektor gemäß Anspruch 2, wobei der Faltkeil (30) derart mit dem zweiten Teilinjektorkörper (5) und/oder mit dem zweiten Teilkolben (7) gekoppelt ist, dass durch ein Verschwenken des proximalen Endes (18) aus dem Lagerungszustand heraus und weg von der Spitzenöffnung (10) der Faltkeil (30) die Intraokularlinse (13) faltet.

5. Injektor gemäß einem der Ansprüche 1 bis 4, wobei der Injektor (1) die Intraokularlinse (13) aufweist, die in dem ersten Injektorkörper (2) und in der Einführrichtung (11) zwischen dem ersten Teilkolben (6) und der Spitzenöffnung (10) angeordnet ist.

6. Injektor gemäß Anspruch 5, wobei der Injektor (1) eingerichtet ist, die Intraokularlinse (13) mit einem Fluid (42) zu benetzen, wenn der Injektorkörper (2) von dem Lagerungszustand in den Gebrauchszustand verschwenkt wird.

7. Injektor gemäß Anspruch 6, wobei der erste Teilinjektorkörper (4) eine Kammer (40) aufweist, in der das Fluid (42) angeordnet ist und die eine Kammeröffnung (43) aufweist, wobei der zweite Teilinjektorkörper (5) und/oder der zweite Teilkolben (7) eingerichtet ist, das Fluid (42) via die Kammeröffnung (43) aus der Kammer (40) zu drücken, indem der Injektorkörper (2) von dem Lagerungszustand in den Gebrauchszustand verschwenkt wird.

8. Injektor gemäß einem der Ansprüche 1 bis 7, wobei der Injektor (1) eine Arretierungsvorrichtung (20) aufweist, die einen Arretierungszustand, in dem die Arretierungsvorrichtung (20) ein Längsverlagern des ersten Teilkolbens (6) und/oder des zweiten Teilkolben (7) arretiert, und einen Entarretierungszustand hat, in dem die Arretierungsvorrichtung (20) das Längsverlagern des ersten Teilkolbens (6) und/oder des zweiten Teilkolbens (7) ermöglicht.

9. Injektor gemäß einem der Ansprüche 1 bis 8, wobei der Injektor (1) eingerichtet ist, dass der zweite Teilkolben (7) den ersten Teilkolben (6) in der Einführrichtung (11) verlagert, wenn der Injektorkörper (2) aus dem Lagerungszustand in den Gebrauchszustand verschwenkt wird.

10. Verpackung mit dem Injektor (1) gemäß einem der Ansprüche 1 bis 9, wobei der Injektor (1) in der Verpackung (50) angeordnet ist und der Injektorkörper (2) in dem Lagerungszustand angeordnet ist.

## Claims

1. Injector for inserting an intraocular lens (13) into the capsular bag of an eye, having an injector body (2), a plunger (3) which is arranged in the injector body (2) so as to be longitudinally displaceable in an insertion direction (11) in which the intraocular lens (13) is to be displaced out of the injector (1) for insertion into the capsular bag, **characterized in that** the injector (1) has a joint (8), wherein the injector body (2) has a first partial injector body (4), which has a tip (9) with a tip opening (10), and a second partial injector body (5), which has a proximal end (18) of the injector body (2) and is pivotably attached to the first partial injector body (4) by means of the joint (8), as a result of which the injector body (2) is pivotable into a use state, in which the proximal end (18) is arranged facing away from the tip opening (10), and a storage state, in which the injector body (2) has a shorter extent in the insertion direction (11) than in the use state, wherein pivoting of the proximal end (18) away from the tip opening (10) makes it possible to bring the injector body (2) from the storage state into the use state, wherein the plunger (3) has a first partial plunger (6), which is arranged on the first partial injector body (4) in a longitudinally displaceable manner, and a second partial plunger (7), which in the storage state is spaced apart from the first partial plunger (6) and in the use state is configured to displace the first partial plunger (6) in the insertion direction (11) by longitudinal displacement of the second partial plunger (7) in the insertion direction (11).

2. Injector according to Claim 1, wherein the injector (1) has a folding wedge (30) which is configured to fold the intraocular lens (13) before the intraocular lens (13) is displaced out of the injector (1).

3. Injector according to Claim 2, wherein the folding wedge (30) is coupled to the second partial injector body (5) and/or to the second partial plunger (7) in such a way that pivoting of the proximal end (18) out of the storage state and towards the tip opening (10) causes the folding wedge (30) to fold the intraocular lens (13).

4. Injector according to Claim 2, wherein the folding wedge (30) is coupled to the second partial injector body (5) and/or to the second partial plunger (7) in such a way that pivoting of the proximal end (18) out of the storage state and away from the tip opening (10) causes the folding wedge (30) to fold the intraocular lens (13).

5. Injector according to any of Claims 1 to 4, wherein the injector (1) has the intraocular lens (13) which is arranged in the first injector body (2) and in the insertion direction (11) between the first partial plunger (6) and the tip opening (10).

6. Injector according to Claim 5, wherein the injector (1) is configured to wet the intraocular lens (13) with a fluid (42) when the injector body (2) is pivoted from the storage state into the use state.

7. Injector according to Claim 6, wherein the first partial injector body (4) has a chamber (40) in which the fluid (42) is arranged and which has a chamber opening (43), wherein the second partial injector body (5) and/or the second partial plunger (7) is configured to push the fluid (42) out of the chamber (40) via the chamber opening (43) by the injector body (2) being pivoted from the storage state into the use state.

8. Injector according to any of Claims 1 to 7, wherein the injector (1) has a locking device (20) which has a locked state, in which the locking device (20) locks a longitudinal displacement of the first partial plunger (6) and/or of the second partial plunger (7), and an unlocked state, in which the locking device (20) allows the longitudinal displacement of the first partial plunger (6) and/or of the second partial plunger (7).

9. Injector according to any of Claims 1 to 8, wherein the injector (1) is configured such that the second partial plunger (7) displaces the first partial plunger (6) in the insertion direction (11) when the injector body (2) is pivoted from the storage state into the use state.

10. Package containing the injector (1) according to any of Claims 1 to 9, wherein the injector (1) is arranged in the package (50) and the injector body (2) is arranged in the storage state.

## Revendications

1. Injecteur pour l'introduction d'une lentille intraoculaire (13) dans le sac capsulaire d'un œil, avec un corps (2) d'injecteur, un piston (3), qui est disposé dans le corps (2) d'injecteur de manière à pouvoir être déplacé longitudinalement dans une direction d'introduction (11), dans laquelle la lentille intraoculaire (13) doit être déplacée hors de l'injecteur (1), **caractérisé en ce que** l'injecteur (1) comporte une articulation (8), le corps (2) d'injecteur comportant un premier corps partiel (4) d'injecteur, qui comporte une pointe (9) avec une ouverture (10) de pointe, et qui comporte un deuxième corps partiel (5) d'injecteur, qui comporte une extrémité proximale (18) du corps (2) d'injecteur et qui est installé sur le premier corps (4) d'injecteur de manière à pouvoir pivoter au moyen de l'articulation (8), ce qui permet au corps (2) d'injecteur de pivoter dans un état d'utilisation, dans lequel l'extrémité proximale (18) de l'ouverture (10) de pointe est disposée de manière opposée, et un état de stockage, dans lequel le corps (2) d'injecteur a une extension plus courte dans la direction d'introduction (11) que dans l'état d'utilisation, un pivotement de l'extrémité proximale (18) loin de l'ouverture (10) de pointe permettant d'amener le corps (2) d'injecteur de l'état de stockage dans l'état d'utilisation, le piston (3) comportant un premier piston partiel (6), qui est disposé de manière à pouvoir être déplacé longitudinalement sur le premier corps partiel (4) d'injecteur, et un deuxième piston partiel (7), qui est disposé dans l'état de stockage de manière espacée du premier piston partiel (6) et est mis au point dans l'état d'utilisation pour déplacer le premier piston partiel (6) dans la direction d'introduction (11) en déplaçant longitudinalement le deuxième piston partiel (7) dans la direction d'introduction (11).

2. Injecteur selon la revendication 1, l'injecteur (1) comportant un coin pliant (30), qui est mis au point pour plier la lentille intraoculaire (13) avant de déplacer la lentille intraoculaire (13) hors de l'injecteur (1).

3. Injecteur selon la revendication 2, le coin pliant (30) étant couplé au deuxième corps partiel (5) d'injecteur et/ou au deuxième piston partiel (7) de telle manière que le coin pliant (30) lie la lentille intraoculaire (18) en faisant pivoter l'extrémité proximale (13) hors de l'état de stockage et en direction de l'ouverture (10) de pointe.

4. Injecteur selon la revendication 2, le coin pliant (30) étant couplé au deuxième corps partiel (5) d'injecteur et/ou au deuxième piston partiel (7) de telle manière que le coin pliant (30) plie la lentille intraoculaire (18) en faisant pivoter l'extrémité proximale (13) hors de l'état de stockage et à l'écart de l'ouverture (10) de pointe.

5. Injecteur selon l'une des revendications 1 à 4, l'injecteur (1) comportant la lentille intraoculaire (13), qui est disposée dans le premier corps (2) d'injecteur et dans la direction d'introduction (11) entre le premier piston partiel (6) et l'ouverture (10) de pointe.

6. Injecteur selon la revendication 5, l'injecteur (1) étant mis au point pour mouiller la lentille intraoculaire (13) avec un fluide (42) lorsque le corps (2) d'injecteur est pivoté depuis l'état de stockage dans l'état d'utilisation.

7. Injecteur selon la revendication 6, le premier corps partiel (4) d'injecteur comportant une chambre (40), dans laquelle le fluide (42) est disposé et qui comporte une ouverture (43) de chambre, le deuxième corps partiel (5) d'injecteur et/ou le deuxième piston partiel (7) étant mis au point pour pousser le fluide (42) hors de la chambre (40) par l'intermédiaire de l'ouverture (43) de chambre en ce que le corps (2) d'injecteur est pivoté depuis l'état de stockage dans l'état d'utilisation.

8. Injecteur selon l'une des revendications 1 à 7, l'injecteur (1) comportant un dispositif de blocage (20), qui a un état de blocage, dans lequel le dispositif de blocage (20) bloque un déplacement longitudinal du premier piston partiel (6) et/ou du deuxième piston partiel (7), et un état de déverrouillage, dans lequel le dispositif de blocage (20) permet le déplacement longitudinal du premier piston partiel (6) et/ou du deuxième piston partiel (7).

9. Injecteur selon l'une des revendications 1 à 8, l'injecteur (1) étant mis au point pour que le deuxième piston partiel (7) déplace le premier piston partiel (6) dans la direction d'introduction (11) lorsque le corps (2) d'injecteur est pivoté depuis l'état de stockage dans l'état d'utilisation.

10. Emballage avec l'injecteur (1) selon l'une des revendications 1 à 9, l'injecteur (1) étant disposé dans l'emballage (50) et le corps (2) d'injecteur étant disposé dans l'état de stockage.
